# EUROPÄISCHE PATENTSCHRIFT

(11) **EP 0 792 625 B1**
(45) Veröffentlichungstag und Bekanntmachung des Hinweises auf die Patenterteilung: **18.07.2001**
(21) Anmeldenummer: 97102506.9
(22) Anmeldetag: 17.02.1997
(51) Int. Cl.: A61B 19/00, A61M 25/10, A61B 5/107

(54) **Vorrichtung und Verfahren zur intraoperativen Kalibrierung einer Manschette bei einer Fundoplicatio**
Device and method for the intraoperative calibration of a sleeve during a fundoplicatio
Dispositif et méthode pour la calibration intra-opérative d'un manchon pendant une fundoplicatio

(30) Priorität: 29.02.1996 DE 19607575
(43) Veröffentlichungstag der Anmeldung: 03.09.1997
(73) Patentinhaber: WILLY RÜSCH AG, D-71394 Kernen-Rommelshausen (DE)
(72) Erfinder: Kalanovic, Daniel, 72074 Tübingen (DE); Haidle, Gerd, 73666 Baltmannsweiler (DE); Roth, Klaus, 72131 Ofterdingen (DE); Kayser, Jaques, 72127 Kusterdingen (DE); Buess, Gerhard, 72074 Tübingen (DE)
(74) Vertreter: KOHLER SCHMID + PARTNER

(56) Entgegenhaltungen:
- FR-A- 790 091
- US-A- 5 338 302
- US-A- 5 352 199
- DATABASE WPI Section PQ, Week 9403 Derwent Publications Ltd., London, GB; Class P31, AN 94-024633 XP002032630 & SU 1 785 663 A (BASHKIR MED INST) , 7.Januar 1993

## Beschreibung

Die Erfindung betrifft eine Vorrichtung und ein Verfahren zur intraoperativen Kalibrierung einer Manschette bei einer Fundoplicatio mit einer Zuführungsleitung, deren körperzugewandtes Ende mit einem druckerfassenden Körper und deren körperabgewandtes Ende mit Mitteln zum Aktivieren des Körpers und zur Messung eines auf den Körper wirkenden Drucks verbindbar ist. Eine Vorrichtung gemäß dem Oberbegriff des Anspruchs 1 ist in SU-A-1 785 663 offenbart.

Eine vergleichbare Vorrichtung ist als Instrument bekannt, das in den Oesophagus eingeführt wird.

Der Speiseröhren-Magenübergang (Kardia) wird außerhalb des Schluckaktes durch das Zusammenwirken der im unteren Bereich des Oesophagus vorhandenen Muskulatur geschlossen. Diese Muskulatur (oesophageale Sphincter) gewährleistet ein System zur Regelung der Nahrungspassage aus der Speiseröhre (Oesophagus) in den Magen. Die Wirkung dieser Regelung beruht auf dem schraubenförmigen Verlauf und der als Sphincter wirksamen horizontalen Einordnung der Muskelfasern im Endteil des Oesophagus sowie auf der Längsspannung des Oesophagus, die beim Schlucken durch eine Muskulaturverkürzung abnimmt.

Wenn nun eine Schwäche der beteiligten Sphincter vorliegt, kann die geöffnete Verbindung von Speiseröhre und Magen außerhalb der Nahrungsaufnahme nicht mehr geschlossen werden, sondern ist stets geöffnet. In diesem Fall liegt eine sogenannte Refluxkrankheit vor, die Reflux-Oesophagitis, die sich in Form von Beschwerden des Patienten bemerkbar macht, wie beispielsweise der Entzündung der Oesophagusschleimhaut, dem Aufstoßen von Säure, Sodbrennen, Schmerzen beim Schlucken, Übelkeit und Brechreiz.

Aus diesem Grund muß die Kardiainsuffizienz entweder internistisch oder operativ behandelt werden, indem beispielsweise eine manschettenförmige Umnähung des Oesophagus mit Fundusfalten erfolgt. Auf diese Weise wird der kardiale Oesophagusabschnitt verengt und ein spitzer Winkel am oesophagogastralen Übergang hergestellt. Die Ausbildung der vernähten Falten in einer Manschettenform dient zur Wiederherstellung des Verschlußmechanismus der Speiseröhren-Magenverbindung.

Nach einem derartigen Eingriff kann die verringerte Öffnung der Speiseröhren-Magenverbindung nun wieder durch die unteren oesophagealen Sphincter geschlossen werden, wobei weder beim Schlucken noch während der temporären Relaxierung ein andauernder minimaler Anpressdruck der Manschette unterschritten wird.

Die Manschette wirkt als "Einbahn"-Ventil, das jeglichen Reflux verhindert. Dies ist das gewünschte Ziel des operativen Eingriffs, das zu Erreichen versucht wird, aber nicht mit Hilfe der bekannten Vorrichtung gelingt, weil intraoperative Druckmessungen im Oesophagus für eine Kalibrierung der Manschette nicht aussagekräftig sind. Schon durch geringste Irritationen der Oesophaguswandungen werden Druckschwankungen auf die bekannte Vorrichtung übertragen. Über die von der Vorrichtung erfaßten Druckschwankungen kann deshalb nicht gesichert auf die Beschaffenheit der Manschette geschlossen werden.

Leider kann es bei nicht richtig ausgebildeter Manschette zu postoperativen Komplikationen kommen, bei denen die am häufigsten auftretenden Symptome Blähungen, Dysphagie und anhaltender Reflux sind. Eine erneute Operation und eine verändert angelegte Manschette ist allerdings nur in 40% aller Fälle möglich, da die miteinander verbundenen Falten im Bereich ihrer vernähten Enden zu starken Vernarbungen neigen, die nur schwer wieder gelöst werden können.

Eine intraoperative Oesophagus-Manometrie erscheint wenig erfolgreich, weil sie die Beschaffenheit der zu einer Manschette vernähten Fundusfalten nur eingeschränkt erfassen kann.

Einen ersten Schritt der Kalibrierung einer Manschette stellt die Verwendung einer bestimmten Manschettengröße dar. Entscheidend ist hierbei, wie fest oder locker die Manschette sitzen muß, um den Speiseröhren-Magenübergang dauerhaft außerhalb des Schluckaktes verschließen zu können. Als unbefriedigende Behandlung der Kardiainsuffizienz wird beispielsweise eine zu weiträumig oder zu tief angelegte Manschette angesehen.

Das Fehlen einer objektiven Kalibrierungsmethode stellt somit noch immer ein Problem dar. Als Kalibrierungsmittel wird in der offenen Chirurgie beispielsweise der in die Manschette eingeführte Zeigefinger des Operateurs verwendet.

Eine bekannte Möglichkeit besteht darin, die Ausbildung der Manschette über die intraoperative Messung des Anpressdrucks auf ein im Bereich der Manschette im Oesophagus angeordnetes Instrument zu erfassen. Bei dieser Methode wird das Instrument in den Oesophagus bis in den Bereich der Manschette eingeführt, dort aktiviert und die Druckbeaufschlagung des Instruments gemessen. Da der gemessene Druck von verschiedenen Faktoren, entscheidend aber von der Lage und Ausrichtung des Instruments im Bereich der Manschette beeinflußt und von Irritationen des Oesophagus überdeckt wird, lassen sich bei der indirekten Messung mit dem bekannten Instrument keine reproduzierbaren und verläßlichen Ergebnisse erzielen. Die Ausbildung der Manschette läßt sich auch durch den Einsatz des bekannten Instruments noch immer nicht standardisieren, weil durch Irritationen des Oesophagus zusätzlich Druckschwankungen auf das Instrument übertragen werden.

Aufgabe der vorliegenden Erfindung ist es daher, eine Vorrichtung und ein Verfahren zu entwickeln, das eine gezieltere Behebung der Kardiainsuffizienz ermöglicht, indem die Beschaffenheit der auf operative Weise ausgebildeten Manschette im Bereich des Speiseröhren-Magenübergangs besser kontrolliert und auf die Stärke der oesophagealen Sphincter abgestimmt werden kann.

Diese Aufgabe wird erfindungsgemäß dadurch gelöst, daß der Körper auf ein vorgegebenes Volumen erweiterbar und bevorzugt als Ballon ausgebildet ist und zumindest im expandierten Zustand eine derartige Außenkontur aufweist, daß eine Anlagefläche gewölbt ausgebildet ist und von zusammengefassten Fundusanteilen unmittelbar übergreifbar ist.

Durch die Schaffung eines Körpers bzw. Ballons, der zwischen dem Oesophagus und der Manschette unmittelbar plaziert werden kann und einen vorgegebenen Raum ausfüllen kann, können Kräfte unmittelbar erfaßt werden, die von der Manschette auf den Körper/Ballon übertragen werden. Der erfaßten Meßwerte können in Relation zum klinischen Bild eines Patienten gesetzt werden.

Durch die gewölbt ausgebildete Anlagefläche und eine seitliche Begrenzung können die die Manschette bildenden Fundusfalten lagefixiert und in stets reproduzierbarer Weise über dem Ballon angeordnet werden. Somit lassen sich verläßliche Meßergebnisse erzielen, die gezielt ausgewertet werden können, und einen Aufschluß über das Zusammenwirken von dem Anpreßdruck auf den Ballon und der Muskelstärke der beteiligten Sphincter ermöglichen. Die Beschaffenheit der aus vernähten Fundusanteilen gebildeten Manschette läßt sich bestimmen und damit kann die Beschaffenheit der Manschette definiert beeinflußt werden.

Der als Ballon ausgebildete Körper ist aus einer dünnen Latex-Lage gefertigt. Der Ballon kann schon geringe Druckschwankungen auf die mit ihm verbundene Meßtechnik übertragen und damit die Wirkung der operativ hergestellten Manschette erfassen. Der aus Latex gefertigte Ballon gewährleistet eine ausreichende mechanische Stabilität. Ein derartiger drucksensibler Schwellkörper ermöglicht die optimale Ausbildung der Manschette unter objektiven Kriterien.

Nach der Ausbildung der Manschette wird der Ballon in die Manschette eingeführt und bis zu einem bestimmten Druck mit einem Gas gefüllt. Anschließend ermöglicht ein Entlüften des Ballons gegebenenfalls eine verbesserte Positionierung. Bei einem erneuten Steigern des Innendrucks des Ballons bis zur Entfaltung des Ballons auf ein vorgegebenes Volumen kann anschließend die Wirkung der Manschette mit dem gemessenen Innendruck korreliert werden. Durch die spezielle Ausbildung der Außenkontur des Ballons wird dabei ein definiertes immer gleiches Meßfeld am Ballon geschaffen. Durch eine definierte Beabstandung der höckerförmigen Erhebungen wird eine reproduzierbare Länge der Nahtreihe der Manschette zur Auswertung herangezogen und die Manschette kann einem ansteigenden Innendruck im Ballon nicht ausweichen. Der Ballon und die höckerförmigen Erhebungen können in verschiedenen Grundgrößen gefertigt sein, so daß schon im Vorfeld der Anwendung die auf den Patienten bestmöglich abgestimmte Vorrichtung ausgewählt werden kann. Bevorzugt wird immer eine Ballongröße bzw. Schwellkörpergröße eingesetzt, die mit einem vorbestimmten Gasvolumen aufgeblasen bzw. auf ein vorbestimmtes Volumen expandiert wird.

Bei einer besonders bevorzugten Ausführungsform umfaßt die Vorrichtung zur Manschettenkalibrierung eine Führungshülse, durch die die Zuführungsleitung in Längsrichtung hindurchgeführt und innerhalb der die Zuführungsleitung verschieblich ist, und bei der ein körperabgewandtes Hülsenende durch eine Verschlußkappe abgedeckt ist. Die Führungshülse und die Verschlußkappe können auch aus einem Stück hergestellt sein.

Bei einer laparoskopischen Fundoplicatio ist der Ballon bei der Einführung in das Abdomen innerhalb der Führungshülse zusammengefaltet angeordnet. Die Führungshülse wird in einen Trokar geschoben, der den Zugang zum Abdomen ermöglicht. Der Ballon wird im Abdomen aus der Führungshülse gedrückt oder geschoben und unter der Manschette aufgeblasen. Ebenso kann der Ballon nach dem Entlüften wiederum in die Führungshülse zurückgezogen werden, so daß die Vorrichtung leicht aus dem Abdomen wieder entfernt werden kann. Daher ist die erfindungsgemäße Vorrichtung zur Manschettenkalibrierung auch zur minimal invasiven Chirurgie geeignet. Dabei wird die Führungshülse mit dem darin befindlichen Ballon über eine Schleuse, beispielsweise einen Trokar, in einen vorbestimmten Körperhohlraum eingeführt.

Bei einer weiteren Ausführungsform weist die Verschlußkappe eine Öffnung zur Durchführung eines Instruments oder dergleichen durch die Führungshülse auf. Die Öffnung kann im nicht verwendeten Zustand durch eine Ventilmembran abgedeckt sein, so daß ein Instrument leicht in die Führungshülse einführbar und die Vorrichtung ansonsten nach außen dicht verschlossen ist. Die Vorrichtung zur Manschettenkalibrierung ermöglicht es, Instrumente zusätzlich zu dem Ballon in den Bauchraum einzuführen, beispielsweise zur Positionierung des Ballons.

Aus Gründen einer sterilen Ausbildung der Vorrichtung ist es vorteilhaft, wenn die Führungshülse aus einem formstabilen Kunststoff und die Verschlußklappe aus Silicon gefertigt sind.

Die oben genannte Aufgabe wird auch durch ein Verfahren gelöst, bei der durch Faltung des Magenfundus um den Oesophagus eine Manschette gebildet wird, die einen auf ein vorbestimmtes Volumen erweiterbaren Körper, bevorzugt einen Ballon umgreift, und der Körper mit Meßinstrumenten in Verbindung steht, die den von der Manschette auf den expandierten Körper unmittelbar ausgeübten Druck erfassen und anzeigen bzw. weiteren Geräten zur Auswertung zuleiten. Nachdem die Beschaffenheit der Manschette über den zwischen Oseophagus und Manschette plazierten expandierten Körper festgestellt und notfalls korrigiert wurde, wird der Körper bezüglich seines Volumens reduziert und aus dem Abdomen entfernt. Der Körper ist bevorzugt ein Ballon aus einem elastischen Material, der das Volumen eines Raumes zwischen Manschette und Oesophagus vorgibt. Erst nachdem der Raum definiert ist, wird der auf den Körper durch die Manschette wirkende Druck erfaßt und ausgewertet.

Als druckerfassende Mittel können Körper beliebiger Art eingesetzt werden, die von einer reduzierten Größe (Volumen) auf eine erweiterte Größe (Volumen) expandierbar sind. Die im erweiterten Zustand des Körpers erfaßten und gemessenen Daten können unmittelbar einer digitalen Auswertung zugeführt werden. Diese Auswertung liefert Ergebnisse über den Istzustand einer Manschette und Daten, die möglicherweise eine Veränderung der Manschette noch nötig machen.

Ein derartiges Verfahren ermöglicht die Erfassung von Krafteinwirkungen der Manschette auf den Ballon in direkter Weise. Wird unter die Manschette in den Hohlraum zum Oesophagus ein Ballon geschoben, der seitlich von zwei höckerförmigen Erhebungen begrenzt ist, so erhält man ein definiertes Meßfeld am Ballon und eine gezielte Messung des Innendrucks des Ballons bei einem bestimmten Innendurchmesser der ausgebildeten Manschette wird ermöglicht.

Aufgrund dieser Korrelation des Balloninnendrucks und des Manschetteninnendurchmessers wird eine gezielte Beseitigung der Kardiainsuffizienz möglich, die erneute operative Eingriffe vermeidet. Die zu einer Manschette zusammengezogenen Falten werden in bekannter Weise verbunden und die erfaßten Meßwerte werden in Relation zum klinischen Bild eines Patienten gesetzt.

Bei einer anderen Variante des Verfahrens wird der Ballon mit Meßinstrumenten über eine Zuführungsleitung verbunden, die von einer Zuführungshülse teilweise umgeben ist. Über bekannte minimal invasive chirurgische Eingriffe kann zunächst ein Zugang zum Bauchraum über einen Trokar geschaffen werden, so daß die Führungshülse mit dem darin aufgenommenen Ballon in den Trokar eingeschoben werden kann und der Ballon im Bereich des Speiseröhren-Magenübergangs positionierbar ist.

Ebenso vorteilhaft ist es, wenn weitere Instrumente durch die Führungshülse in den Körperhohlraum eingeführt werden, um die Positionierung des eingeführten Ballons unterstützen zu können.

Besonders vorteilhaft ist es, wenn die Manschette auf einer konvex ausgebildeten Anlagefläche des Ballons aufliegt und die diametral zur Anlagefläche ausgebildete Fläche des Ballons konkav ausgebildet ist und auf der Außenwandung des Oesophagus plaziert wird. Damit läßt sich der erfindungsgemäße Ballon sicher positionieren und die Manschette kann sich formschlüssig um den Ballon legen. Die konkav ausgebildete Fläche des Ballons ist bevorzugt einem im Oesophagus plazierten Instrument angepaßt, der den Oesophagus im Bereich der Manschette schient. Druckschwankungen bzw. der Anpreßdruck der Manschette können vom Ballon besser erfaßt und weitergeleitet werden, weil der Katheter die Funktion eines Widerlagers übernimmt.

Bei der laparoskopischen Fundoplicatio ist der Ballon in einer Führungshülse untergebracht und die Führungshülse wird über einen Zugang in das Abdomen eingeführt. Bevorzugt wird der Zugang von einem Trokar gebildet. Im Abdomen wird der Ballon aus der Führungshülse herausgeschoben bzw. herausgezogen und unter der Manschette wird der Ballon aufgeblasen.

Besondere Vorteile ergeben sich bei der laparoskopischen Fundoplicatio, wenn die Manschette zwischen den farblich vom übrigen Ballon abgesetzten seitlichen Erhebungen vernäht wird. Über den farblichen Kontrast von den seitlichen Erhebungen zum übrigen Ballon lassen sich Abstände sicherer abschätzen bzw. bestimmen.

Mit dem erfindungsgemäßen Verfahren und der dazu geschaffenen Vorrichtung läßt sich eine intraoperative Kalibrierung einer Manschette sowohl in offener Chirurgie als auch bei einem laparoskopischen Eingriff unter objektiven und wiederholbaren Kriterien erreichen, die nicht von Sphincterirritationen überdeckt werden.

Weitere Vorteile ergeben sich aus der Beschreibung der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Die Erfindung ist in der Zeichnung dargestellt und wird anhand eines Ausführungsbeispieles näher erläutert. Es zeigt:
- Fig. 1: eine Vorrichtung zur Manschettenkalibrierung mit einer Führungshülse, in der ein nicht aktivierter Ballon untergebracht ist;
- Fig. 2: die Vorrichtung nach Fig. 1 mit dem außerhalb der Führungshülse aktivierten Ballon;
- Fig. 3: den aktivierten Ballon nach Fig. 2, der von einer Manschette aus Fundusfalten umschlossen ist.

Die Figuren der Zeichnung zeigen den erfindungsgemäßen Gegenstand stark schematisiert und sind nicht notwendigerweise maßstäblich zu verstehen. Die gegenständlichen Merkmale sind in den einzelnen Figuren so dargestellt, daß der Aufbau der Vorrichtung und die Formgebung des Ballons gut gezeigt werden kann.

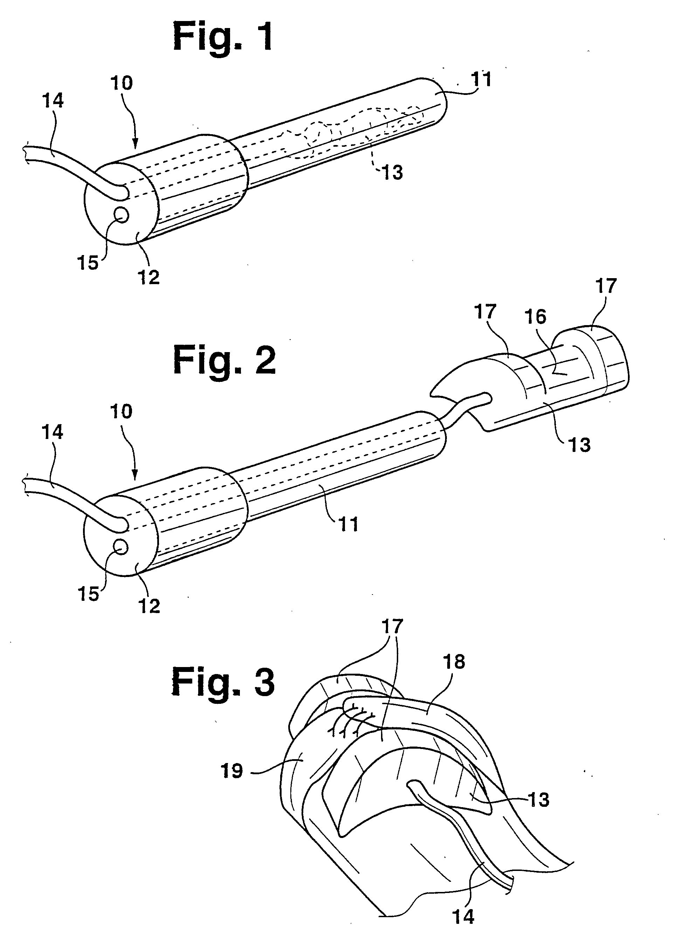

In Fig. 1 ist eine Vorrichtung zur Manschettenkalibrierung 10 perspektivisch dargestellt, wie sie bei einer laparoskopischen Fundoplicatio Verwendung finden kann. Die Vorrichtung 10 besteht aus einer Führungshülse 11, die einenends durch eine Verschlußkappe 12 abgedeckt ist und anderenends offen ausgebildet ist. Die Verschlußkappe 12 kann aus einem Silicon gefertigt sein und die Führungshülse 11 dicht umschließen. Die Verschlußkappe 12 und die Führungshülse 11 kann in einer weiteren Ausführungsform einstückig ausgebildet sein. Die Vorrichtung 10 ist dazu geeignet, in die bei endoskopischen Operationen verwendeten Trokare, bevorzugt 10-12 mm Trokare, eingeführt zu werden. Innerhalb der Führungshülse 11 ist ein Ballon 13 untergebracht, der mit einer Zuführungsleitung 14 verbunden ist, über die der Ballon 13 über in der Figur nicht sichtbare Hilfsmittel erstens aus der Führungshülse 11 herausgeschoben und aufgeblasen werden kann. Wenn der Ballon 13 vollständig innerhalb der Führungshülse 11 untergebracht ist, wird durch die Vorrichtung 10 ein durch den Ballon 13 gebildeter möglicher Einführwiderstand über den Trokar in den Bauchraum verhindert. Weiterhin weist die Vorrichtung 10 eine abgedichtete Öffnung 15 auf, durch die Mittel zur Unterstützung der Positionierung des Ballons 13 einführbar sind, oder Operationsinstrumente für andere Aufgaben.

In der Fig. 2 ist die Vorrichtung 10 nach dem Austritt des Ballons 13 aus der Führungshülse 11 im aufgeblasenen Zustand des Ballons 13 gezeigt. Der Ballon 13 ist über die Zuführungsleitung 14 mit Mitteln verbunden, die ein Aufblasen oder Entlüften des Ballons 13 ermöglichen. Dabei ist der Ballon 13 gezielt mit einem bestimmten vorgebbaren und meßbaren Balloninnendruck aufblasbar.

Zur exakten Positionierung im Bereich des Speiseröhren-Magenübergangs weist der Ballon 13 im aufgeblasenen Zustand eine charakteristische erfindungsgemäße Außenkontur auf. Die Ausbildung dieser Außenkontur gewährleistet ein immer gleiches Meßfeld. Im aufgeblasenen Zustand weist der Ballon 13 eine gewölbte Anlagefläche 16 auf, an der die aus Fundusfalten gebildete Manschette zur Anlage kommt. Diametral zur Anlagefläche 16 ist der Ballon 13 mit einer konkaven Fläche ausgebildet, die sich besonders gut an die Außenoberfläche des Oesophagus im Bereich des Speiseröhren-Magenübergangs anschmiegen kann. Die Anlagefläche 16 ist seitlich von höckerförmigen Erhebungen 17 begrenzt, so daß der Ballon 13 durch die ihn übergreifende Manschette lagefixiert ist. Dies ist auch möglich, wenn nur ein geringer Innendruck innerhalb des Ballons 13 vorhanden ist. Der Ballon 13 ist aus einem hochelastischen, gewebeverträglichen und widerstandsfähigen Material, z.B. Latex, hergestellt.

Fig. 3 zeigt eine erfindungsgemäße Verwendung der Vorrichtung 10 in perspektivischer Darstellung. Die Vorrichtung 10 ist vergrößert und nur teilweise sichtbar dargestellt. In der Figur sind der Ballon 13 und seine Zuführungsleitung 14 zu erkennen. Nach einer Fundoplicatio sind Fundusfalten 18 und 19 (Fundusteile) zusammengezogen worden, um eine Manschette zu bilden, die die Öffnung des Speiseröhren-Magenübergangs verringert. Dies stellt eine operative Maßnahme dar, um eine Kardiainsuffizienz zu beheben.

Da der Ballon 13 im aufgeblasenen Zustand Erhebungen 17 aufweist, kann die durch die Fundusfalten 18 und 19 gebildete Manschette um den Ballon 13 auf die in der Figur verdeckte Anlagefläche 16 gelegt und dort lagefixiert werden. Die Erhebungen 17 sind vom übrigen Ballon 13 farblich abgesetzt.

Über die Begrenzung einer Fläche durch Erhebungen 17 wird ein immer gleiches Meßfeld des Ballons 13 bereitgestellt, um eine Korrelation des Balloninnendrucks mit der Stärke der durch die vorzugsweise vernähten Fundusfalten 18 und 19 gebildeten Manschette zu messen. Nach Kenntnis einer derartigen Korrelation kann die Beschaffenheit der ausgebildeten Manschette intraoperativ geprüft und notfalls auf gewünschte Beschaffenheitswerte korrigiert werden.

An der Zuleitung 14 können verschiedenste Manometer angeschlossen werden, über die auch geringste Druckschwankungen im Ballonbereich erfaßt und ausgewertet werden können. Ebenfalls kann die Zuführungsleitung 14 geteilt sein, so daß über diese Zuführungsleitung 14 einerseits der Ballon aufgeblasen werden kann und durch Umstellung eines Ventils wird die Zuführungsleitung 14 auf ein Druckerfassungs- bzw. -anzeigegerät geleitet.

Eine Vorrichtung (10) zur intraoperativen Kalibrierung einer Manschette bei einer Fundoplicatio weist eine Zuführungsleitung (14) auf, deren körperzugewandtes Ende mit einem aufblasbaren Ballon (13) und deren körperabgewandtes Ende mit Mitteln zum Aufblasen/Entlüften des Ballons (13) und zur Messung des Innendruckes des Ballons (13) verbindbar ist. Der Ballon (13) weist zumindest im aufgeblasenen Zustand eine derartige Außenkontur auf, daß eine Anlagefläche (16) gewölbt ausgebildet und seitlich durch zwei höckerförmige Erhebungen (17) begrenzt ist. Fundusanteile werden oesophagusumgreifend zu einer Manschette zusammengefaßt und die Beschaffenheit der Manschette wird über von der Manschette auf den Ballon ausgeübte Kräfte bestimmt.

## Patentansprüche

1. Vorrichtung (10) zur intraoperativen Kalibrierung einer Manschette bei einer Fundoplicatio, mit einer Zuführungsleitung (14), deren körperzugewandtes Ende mit einem druckerfassenden, auf ein vorgegebenes Volumen zur Definition eines Raumes erweiterbaren Ballon (13) und deren körperabgewandtes Ende mit Mitteln zum Aktivieren des Ballons (13) und zur Messung eines auf den Ballon (13) wirkenden Drucks verbindbar ist, wobei der Ballon zumindest im expandierten Zustand eine derartige Außenkontur aufweist, dass eine Anlagefläche (16) gewölbt ausgebildet und von zusammengefassten Fundusanteilen unmittelbar übergreifbar ist, dadurch gekennzeichnet, daß die Anlagefläche seitlich durch zwei höckerförmige Erhebungen (17) begrenzt ist.

2. Vorrichtung nach Anspruch 1, dadurch gekennzeichnet, dass die Vorrichtung (10) zur Kalibrierung einer Manschette eine Führungshülse (11) umfasst, durch die die Zuführungsleitung (14) in Längsrichtung hindurchgeführt und nnerhalb der die Zuführungsleitung (14) verschieblich st, und dass ein körperabgewandtes Hülsenende durch eine Verschlusskappe (12) abgedeckt ist.

3. Vorrichtung nach Anspruch 2, dadurch gekennzeichnet, dass die Verschlusskappe (12) eine Öffnung (15) zur Durchführung eines Instruments oder dergleichen durch die Führungshülse (11) aufweist.

## Claims

1. Device (10) for calibration of a collar during a fundoplicatio, comprising a supply line (14) whose end facing the body can be connected to a pressure-detecting balloon (13) which can be expanded to a predetermined volume for defining a space, and whose end facing away from the body can be connected to means for activating the balloon (13) and measuring of a pressure acting on the balloon (13), wherein the balloon has, at least in the expanded state, such an outer shape that an abutment surface (16) is curved and can be directly overlapped by the combined fundus parts, characterized in that the abutment surface is delimited on the side through two hump-shaped elevations (17).

2. Device according to claim 1, characterized in that the device (10) for calibrating a collar comprises a guiding sleeve (11) through which the supply line (14) is guided in the longitudinal direction and within which the supply line (14) can be displaced and that a sleeve end facing away from the body is covered by a closing cap (12).

3. Device according to claim 2, characterized in that the closing cap (12) comprises an opening for passage of an instrument or the like through the guiding sleeve (11).

## Revendications

1. Dispositif (10) pour le calibrage intra-opératoire d'un manchon pendant une fundoplicatio, comportant une conduite d'alimentation (14) dont l'extrémité tournée vers le corps peut être reliée à un ballon (13) détectant la pression et pouvant être dilaté à un volume donné pour définir un espace et dont l'extrémité détournée du corps peut être reliée à des moyens pour activer le ballon (13) et pour mesurer une pression agissant sur le ballon (13), le ballon présentant du moins dans l'état dilaté un contour extérieur tel qu'il se forme une surface d'appui (16) bombée et susceptible d'être coiffée directement par des portions regroupées du fundus, caractérisé en ce que la surface d'appui est délimitée latéralement par deux surélévations (17) en forme de bosse.

2. Dispositif selon la revendication 1, caractérisé en ce que le dispositif (10) destiné au calibrage d'un manchon comprend une douille de guidage (11) à travers laquelle est menée la conduite d'amenée (14) en direction longitudinale et à l'intérieur de laquelle la conduite d'amenée (14) est mobile, et en ce qu'une extrémité de la douille détournée du corps est recouverte par un capuchon d'obturation (12).

3. Dispositif selon la revendication 2, caractérisé en ce que le capuchon d'obturation (12) présente une ouverture (15) pour faire passer un instrument ou similaire à travers la douille de guidage (11).
